# EUROPEAN PATENT APPLICATION

(11) **EP 3 653 204 A1**
(43) Date of publication of application: **20.05.2020**
(21) Application number: 19217776.4
(22) Date of filing: 18.01.2011
(51) Int. Cl.: A61K 9/48, A61K 31/4545, A61K 9/107

(54) **PHARMACEUTICAL FORMULATIONS OF LORATADINE FOR ENCAPSULATION AND COMBINATIONS THEREOF**

(30) Priority: 19.01.2010 CA 2690490
(62) Divisional of application: 11734280.8
(71) Applicant: Catalent Ontario Limited, Windsor, ON N9C 3R5 (CA)
(72) Inventor: OKUTAN, Beth, London, Ontario ON N6G 4R1 (CA); DRAPER, Peter, LaSalle, Ontario N9J 3E9 (CA); DRAPER, James, LaSalle, Ontario N9J 1J9 (CA)
(74) Representative: Maiwald Patent- und Rechtsanwaltsgesellschaft mbH

(57) **Abstract**

This invention relates to a loratadine formulation suitable for encapsulation into a soft gel capsule or suitable dosage unit with improved functionality providing enhanced *in vitro* dissolution and bioavailability of loratadine. The invention also provides a formulation with improved functionality as a highly concentrated solution within a given fill volume in order to manufacture as small a capsule as possible to facilitate consumer acceptance and acceptable manufacturing costs. The invention also relates to a formulation of optimal stability suitable for supporting a fill composition compatible with the soft gel capsule dosage unit.

## Description

### FIELD OF THE INVENTION

The present invention generally relates to oral pharmaceutical formulations. More particularly, the present invention relates to an improved pharmaceutical formulation of loratadine suitable for encapsulation into a soft gel capsule or other suitable dosage unit.

### BACKGROUND OF THE INVENTION

Two identified histamine receptors are the receptors H-1 and H-2. The H-1 receptors mediate the response antagonized by conventional antihistamines. H-1 receptors are present in the mammalian skin, ileum and bronchial smooth muscle.

Non-narcotic or non-sedating hydrophobic antihistamine compounds, such as loratadine, are known. Loratadine was described, for example, in United States Patent No. 4,282,233 to Vilani. Loratadine is an H-1 histamine receptor protein antagonist which binds to peripheral H-1 receptors, as discussed in Quercia et al., Hosp. Formul., 28, p.137-53 (1993). Loratadine is useful as an antihistamine and has little or no sedative effects. Thus, loratadine provides an antihistamine effect while still allowing the user to perform mental or physical functions requiring high levels of concentration. Other therapeutic treatments using loratadine alone or in combination with other active ingredients have been suggested, such as treatment of seasonal or perennial rhinitis, allergic asthma, and motion sickness. See Aberg et al., United States Patent No. 5,731,319, for example. Antiarrhythmic uses, such as treatment of atrial fibrillation (AF), have also been suggested, as described in Buckland et al., United States Patent No. 6,110,927.

Loratadine derivatives that share antihistamine properties of loratadine have also been developed. Active metabolites such as decarbalkoxylated forms of loratadine have been of interest. One such metabolite derivative is 8-chloro-6,11-dihydro-11-(4-piperidylidine)-5H-benzo-[5,6]-cyclohepta-[1,2-b] pyridine, also known as descarboethoxyloratadine (DCL) which is described in United States Patent No. 4,659,716 to Villani. United States Patent No. 5,595,997 to Aberg et al discloses methods of utilizing DCL for the treatment of allergic rhinitis and other disorders without adverse side effects.

Other patents relating to loratadine or a chemically related antihistamine, including any pharmaceutically acceptable salt thereof, in various dosage forms include United States Patent No. 5,100,675 to Cho et al.; United States Patent No. 4,990,535 to Cho et al.; and United States Patent No. 5,314,697 to Kwan et al.

Oral dosage forms, such as loratadine-containing tablets and syrups, are known and marketed under the names Claritin®, Claritin Reditabs® and Claritin-D® 24-Hour etc. (commercially available from Schering-Plough Corporation, NJ).

These commercial products are described in United States Patent Nos. 4,282,233 to Villani; 4,659,716 to Villani; 4,863,931 to Schumacher et al.; and 6,132,758 to Munayyer et al. United States Patent No. 6,132,758 discloses an antihistaminic syrup stabilized against degradation of the active ingredient by the addition of about 0.05 to 5 mg/mL of an amino-polycarboxylic acid. This patent teaches that, under certain storage conditions, losses of active agent can occur.

United States Patent No. 4,910,205 to Kogan et al. discloses a transdermally acceptable composition comprising an effective amount of loratadine or its decarbalkoxylation product, about 40-70% weight % of a volatile solvent, about 5-50% by weight of a fatty acid ester and about 2-60% of an essential oil.

While syrup, solid and fast dissolving dosage forms are available for loratadine type antihistamines, soft gel capsule dosage forms have proven more difficult to formulate and market. The soft gel capsule dosage form has many advantages known to those skilled in the art. Oral delivery systems for hydrophobic drugs suitable for encapsulation in gelatin dosage forms are described in United States Patent No. 6,096,338 to Lacy et al. However, formulating hydrophobic drugs, such as loratadine, into solutions for encapsulation into a soft gel capsule dosage form can present many problems.

Compounds such as loratadine are susceptible to recrystallization. Thus, hydrophobic solvents are preferred for use in soft gel capsule dosage forms so as to reduce the hydrophilic nature of the fill, which can cause recrystallization and precipitation of the active ingredient under storage conditions. Ideally, a solvent system for loratadine is one which is hydrophobic, protonic and water-dispersible. One problem associated with hydrophobic solvents, however, is that they are known to adversely affect bioavailability of the drug.

A further limitation in the use of soft gel capsule dosage forms is that it may not be possible to dissolve the desired amount of pharmaceutical agent in a volume of solvent small enough to produce a soft gel capsule dosage form which delivers the desired dosage amount, is economically appropriate and comfortable to ingest by the patient.

Several formulations have been developed in efforts to increase the solubility, long-term storage stability and the bioavailability of loratadine and its derivatives. Several such prior art formulations are disclosed by Lin et al. in United States Patent No. 7,201,921 and United States Patent No. 6,720,002, both of which disclose the use of a solvent system comprising a mixture of medium chain fatty acids for enhancing the solubility of decarbalkoxylated loratadine compositions, to produce a highly concentrated solution of the loratadine derivative suitable for encapsulation in soft gel capsules. The solvent system includes a mixture of mono- and diglycerides of medium chain fatty acids, and provides for a highly concentrated solution capable of encapsulation into a small vessel, such as a soft gel capsule, to permit easy swallowing and to provide a pharmaceutically effective dose of loratadine compositions.

The formulations disclosed in the Lin et al. references are said to provide loratadine compositions exhibiting good solubility and storage stability while maintaining bioavailability of the drug. The loratadine formulations of the Lin et al. references include loratadine compositions comprised of either decarbalkoxylated loratadine derivatives, or a mixture of loratadine and loratadine derivatives. The references do not, however, specifically address the problems associated with storage stability and recrystallization of fill compositions solely comprised of loratadine for encapsulation in soft capsule dosage forms.

Increasing the quantity of loratadine in soft gel capsule dosage forms without necessitating an increase in overall fill volume (and thereby increasing overall size of the dosage form) and/or without increasing instability of loratadine and without the use of loratadine derivatives has proven difficult to accomplish in the art.

There thus exists a need for improved pharmaceutical formulations containing loratadine for use in soft gel capsule dosage forms which solubilizes loratadine and exhibits long-term storage stability at ambient conditions without recrystallization. Increased *in vitro* dissolution of loratadine effective faster absorption is desirable. There is also a need for a fill formulation which does not adversely affect bioavailability of the active ingredient. Also desirable is a formulation which satisfies both of these criteria and also increases the fill concentration of loratadine. This would permit the use of smaller size capsules for a given dose of active.

It would be advantageous and desirable to have improved pharmaceutical formulations of loratadine which are capable of supporting loratadine concentrations sufficient for encapsulation in a soft gel capsule dosage forms of appropriate size and with acceptable manufacture economics.

### SUMMARY OF THE INVENTION

In accordance with an aspect of the present invention, there is provided a pharmaceutical formulation comprising loratadine, one or more mono- and di- glyceride medium chain fatty acids, one or more dispersants and one or more surfactants for enhancing *in vitro* dissolution and bioavailability of loratadine. There is also provided a pharmaceutical formulation comprising loratadine, a mixture of one or more mono- di-triglyceride medium chain fatty acids and one or more triglyceride medium chain fatty acids, one or more dispersants and one or more surfactants for enhancing *in vitro* dissolution and bioavailability of loratadine.

In accordance with another aspect of the present invention, there is provided a loratadine dosage form for enhancing *in vitro* dissolution and bioavailability of loratadine comprising a drug delivery vehicle and a formulation comprising loratadine, one or more mono- and di- glyceride medium chain fatty acids, one or more dispersants and one or more surfactants disposed within the drug delivery vehicle. There is also provided a loratadine dosage form for enhancing *in vitro* dissolution and bioavailability of loratadine comprising a drug delivery vehicle and a formulation comprising loratadine, a mixture of one or more mono- di- triglyceride medium chain fatty acids and one or more triglyceride medium chain fatty acids, one or more dispersants and one or more surfactants disposed within the drug delivery vehicle.

It is a further aspect of the present invention to produce a formulation with improved functionality as a highly concentrated solution within a given fill volume in order to manufacture as small a capsule as possible to facilitate consumer acceptance and acceptable manufacturing costs.

The invention further provides a formulation of optimal stability suitable for supporting a fill composition compatible within soft gelatin capsules or suitable dosage forms.

### DETAILED DESCRIPTION OF THE PREFERRED EMBODIMENTS

An improved pharmaceutical formulation capable of supporting higher loratadine concentrations, having enhanced *in vitro* dissolution and bioavailability and having acceptable stability suitable for encapsulation in soft gelatin capsules or suitable dosage forms is disclosed. Also disclosed is a pharmaceutical formulation suitable for combination with other active pharmaceutical ingredients. The loratadine formulation of the present invention generally includes loratadine, mono- and di-glyceride medium chain fatty acids, dispersant and surfactant. The loratadine formulation may further comprise triglyceride medium chain fatty acids. Additionally, water may be included as required.

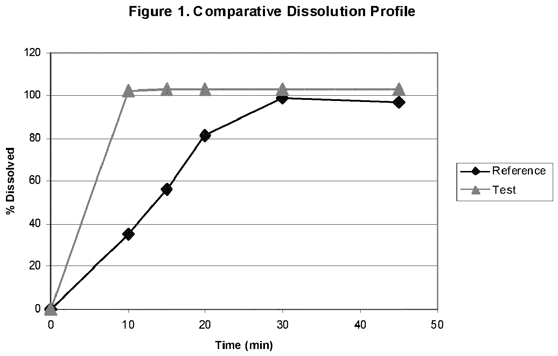

In Figure 1, comparative dissolution profiles between a formulation of the present invention and a reference product, commercially available Claritin® Liqui-Gels®, are shown. The test dissolution profile is for the formulation disclosed in Example 6 and conducted in accordance with procedure described in Example 23. Figure 1 shows improved *in vitro* dissolution for the formulation of the present invention as compared to the reference product. Thus, the formulation of the present invention releases loratadine faster *in vitro* than the reference product.

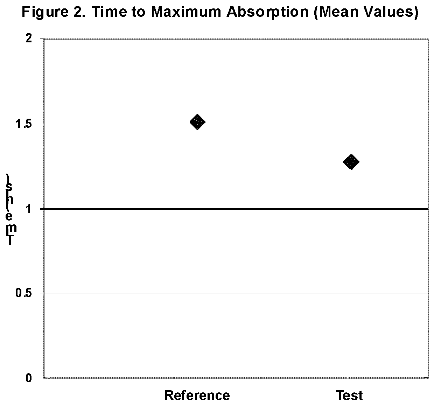

Figure 2 shows the time to maximum concentration of loratadine in blood plasma in man for a test formulation of the present invention disclosed in Example 6 and a reference product, commercially available Claritin® Liqui-Gels®. Figure 2 shows that loratadine formulated according to the present invention is absorbed approximately 18% faster into the blood stream than the reference product.

The loratadine formulation of the invention produces a solution that accommodates higher concentrations of the active ingredient suitable for encapsulation in soft gel capsules or suitable dosage forms, without needing to use loratadine derivatives, such as decarbalkoxylated forms of loratadine. The elimination of the decarbalkoxylated forms of loratadine permits a more economical manufacturing process.

The loratadine compounds of the invention can be prepared according to the method described in United States Patent No. 4,282,233 to Villani. The starting materials and reagents to prepare loratadine are well known in the art and readily available, and loratadine can be synthesized using conventional organic synthesis techniques. Any suitable pharmaceutical grade of loratadine may be used in the formulation described herein. The amount of loratadine in the formulation ranges from about 3% to about 7% by weight.

Functional fill formulations which can be used in accordance with the invention are those which are both moderately lipophilic and have hydrogen bonding capability. Preferably, the formulation has a hydrophilic lipophilic balance (HLB) value ranging from about 3 to about 7, more preferably ranging from about 5 to about 6. Preferably, the formulation has a mixture of monoglyceride medium fatty acids (with HLB value ranging from about 5 to about 6) and triglyceride medium chain fatty acids (with an HLB value of about 11) as a functional solubilizer.

Suitable functional fill formulations may comprise one or more mono- and di-medium chain fatty acids. Suitable mono and di-glyceride medium chain fatty acids include, but are not limited to, glyceryl mono- & dicaprate (such as CAPMUL™ MCM available from Abitec Corporation), propylene glycol monocaprylate (such as CAPRYOL™ 90 available from Gattefosse), propylene glycol caprylate (such as CAPRYOL™ PGMC available from Gattefosse), propylene glycol laurate (LAUROGLYCOL™ FCC available from Gattefosse) and propylene glycol monolaurate (LAUROGLYCOL™ 90 available from Gattefosse). The mono- and di- glyceride medium fatty chain acids may be present in the formulation in a total amount from about 33% to about 83% by weight.

Suitable functional fill formulations may also comprise a mixture of one or more mono- and di- triglyceride medium chain fatty acids and one or more triglyceride medium chain fatty acids. Suitable triglyceride medium chain fatty acids include, but are not limited to, glyceryl tricaprylate/caprate (such as CAPTEX™ 300 available from Abitec Corporation), glycerol caprylate caprate (such as CAPTEX™ 355 available from Abitec Corporation), and caprylic/capric triglyceride (such as LABRAFAC™ CC available from Gattefosse, MIGLYOL™ 810 available from Sasol or MIGLYOL™ 812 available from Sasol). Most preferred are the monoglyceride medium chain fatty acid CAPMUL™ MCM and triglyceride medium chain fatty acid CAPTEX™ 355. The triglyceride medium fatty chain acids may be present in the formulation in a total amount from about 8% to about 50% by weight.

The functional fill formulations of the present invention can further comprise a dispersant composition of one or more dispersants to enhance uniform dispersibility of the fill in water. The amount of the additional dispersant, however, is present in amount sufficient to enhance uniform dispersion of the fill in water or gastric juices without significantly increasing the volume of the fill. The dispersants may be present in a total amount from about 7% to about 12% by weight. Suitable dispersants which can be used include, but are not limited to, povidone, such as K-12, K-17 or K-30. The dispersant composition used in accordance with the invention can be a combination of povidone together with one or more surfactants. A preferred dispersant is a mixture of povidone K-12 and Polysorbate™ 80 present in a ratio of about 1.75:1 to about 2.5:1).

Suitable surfactants which can be used include, but are not limited to, non-ionic surfactants having an hydrophilic lipophilic balance value ranging from about 14 to about 17; polyoxyethylene sorbitan fatty acid esters, such as Polysorbate™ 40, Polysorbate™ 80, Polysorbate™ 60, Polysorbate™ 20, and Polysorbate™ 120; ethoxylated aliphatic alcohols, such as Oleth-20 (Volpo™ 20 available from Croda, Inc., Parsippany, N.J.), Ceteareth-20 (Volpo™ CS-20 available from Croda, Parsippany, N.J.); and caprylocaproyl macrogol-8 glycerides (LAUROGLYCOL™ 90 available from Gattefosse). The surfactants may be present in an aggregate amount from about 2% to about 5% by weight.

The functional fill formulations of the present invention may also comprise water in an amount from about 1% to about 4% by weight.

Soft gel capsules containing pharmaceutical compositions can be prepared using conventional and known encapsulation techniques, such as that described in Stroud et al., United States Patent No. 5,735,105. In general, gel capsule formulations for soft gel capsule dosage forms consist of raw gelatin and one or more ingredients which are added to plasticize the gelatin to produce a capsule to suitable hardness as required by design or by preference. Those skilled in the art will appreciate what capsule material compositions are suitable.

Suitable plasticizers include the materials used for the same purpose in the manufacture of mammalian gelatin capsules. Representative plasticizers are any of a variety of polyhydric alcohols such as glycerin, sorbitol, propylene glycol, polyethylene glycol and the like. Other plasticizers can include saccharides and polysaccharides, which can be prepared by hydrolysis and/or hydrogenation of simple or complex polysaccharides.

Other components can also be incorporated into the gelatin capsule compositions provided they do not alter the melting point/fusion point characteristics of the film. Representative of these additional components, typically added during the molten state of the capsule composition, include flavoring agents, opacifying agents, preservatives, embrittlement inhibiting agents, colorants, dyes and pigments, and disintegrants. Use of conventional pharmaceutical or food grade ingredients for functional, organoleptic and appearance purposes are acceptable provided they do not alter the characteristics of the film and functional characteristics of the soft gel capsule.

Soft gel capsule dosage forms containing the loratadine compositions of the invention can be orally administered to patients in need of H1 receptor antagonist or antihistamine treatment.

When formulated in accordance with the present invention, a 10 mg loratadine dose can be accommodated by a 5 minim or less size oval soft gel capsule. A 10 mg loratadine dose can be contained within a capsule size as small as a 2.3 minim size oval soft gel capsule. Capsule size volumes of the present invention are expressed in terms of minims, wherein 1 minim = 0.0616 cc.

Pro-rata adjustments may be made to the formulation of the present invention to accommodate other dosages, such as 5, 15 and 20 mg loratadine. The formulations of the present invention are suitable for filling such dosages into appropriate size and shape soft gel capsules or suitable dosage forms. Inherent in the manufacturing process is the capability to manufacture capsules over a wide range of shapes and sizes to accommodate the required amount of fill material.

Below are examples illustrating several loratadine formulations made in accordance with the present invention. The examples presented below are intended to illustrate particular embodiments of the invention and are not intended to limit the scope of the specification, including the claims, in any way.

### EXAMPLES

### Loratadine Soft Gel Capsule Fill Formulations - Per Soft Gel Capsule

### EXAMPLE 1:

| **Ingredient** | **(%w/w)** | **mg** |
|---|---|---|
| Loratadine | 6.250 | 10 |
| Captex™ 355 | 40.625 | 65 |
| Capmul™ MCM | 40.625 | 65 |
| Polysorbate™ 80 | 3.125 | 5 |
| Povidone K-12 | 6.250 | 10 |
| Water | 3.125 | 5 |
| Total | 100.000 | 160 |

### EXAMPLE 2:

| **Ingredient** | **(%w/w)** | **mg** |
|---|---|---|
| Loratadine | 6.250 | 10 |
| Captex™ 300 | 40.625 | 65 |
| Capmul™ MCM | 40.625 | 65 |
| Polysorbate™ 80 | 3.125 | 5 |
| Povidone K-12 | 6.250 | 10 |
| Water | 3.125 | 5 |
| Total | 100.000 | 160 |

### EXAMPLE 3:

| **Ingredient** | **(%w/w)** | **mg** |
|---|---|---|
| Loratadine | 6.250 | 10 |
| Captex™ 355 | 41.188 | 65.9 |
| Capmul™ MCM | 41.188 | 65.9 |
| Polysorbate™ 80 | 3.125 | 5 |
| Povidone K-17 | 6.250 | 10 |
| Water | 1.999 | 3.2 |
| Total | 100.000 | 160 |

### EXAMPLE 4:

| **Ingredient** | **(%w/w)** | **mg** |
|---|---|---|
| Loratadine | 6.250 | 10 |
| Captex™ 300 | 40.625 | 65 |
| Capmul™ MCM | 40.625 | 65 |
| Polysorbate™ 80 | 3.125 | 5 |
| Povidone K-17 | 6.250 | 10 |
| Water | 3.125 | 5 |
| Total | 100.000 | 160 |

### EXAMPLE 5:

| **Ingredient** | **(%w/w)** | **mg** |
|---|---|---|
| Loratadine | 6.250 | 10 |
| Captex™ 300 | 40.625 | 65 |
| Capmul™ MCM | 40.625 | 65 |
| Polysorbate™ 80 | 3.125 | 5 |
| Povidone K-30 | 6.250 | 10 |
| Water | 3.125 | 5 |
| Total | 100.000 | 160 |

### EXAMPLE 6:

| **Ingredient** | **(%w/w)** | **mg** |
|---|---|---|
| Loratadine | 6.3 | 10 |
| Captex™ 355 | 41.2 | 65.9 |
| Capmul™ MCM | 41.2 | 65.9 |
| Polysorbate™ 80 | 3.1 | 5 |
| Povidone K-12 | 6.3 | 10 |
| Water | 2.0 | 3.2 |
| Total | 100 | 160 |

### EXAMPLE 7:

| **Ingredient** | **(%w/w)** | **mg** |
|---|---|---|
| Loratadine | 6.3 | 10 |
| Captex™ 355 | 49.4 | 79.1 |
| Capmul™ MCM | 33.0 | 52.7 |
| Polysorbate™ 80 | 3.1 | 5 |
| Povidone K-12 | 6.3 | 10 |
| Water | 2.0 | 3.2 |
| Total | 100 | 160 |

### EXAMPLE 8:

| **Ingredient** | **(%w/w)** | **mg** |
|---|---|---|
| Loratadine | 6.3 | 10 |
| Captex™ 355 | 20.6 | 33 |
| Capmul™ MCM | 61.8 | 98.9 |
| Polysorbate™ 80 | 3.1 | 5 |
| Povidone K-12 | 6.3 | 10 |
| Water | 2.0 | 3.2 |
| Total | 100 | 160 |

### EXAMPLE 9:

| **Ingredient** | **(%w/w)** | **mg** |
|---|---|---|
| Loratadine | 6.3 | 10 |
| Captex™ 355 | 8.2 | 13.2 |
| Capmul™ MCM | 74.1 | 118.6 |
| Polysorbate™ 80 | 3.1 | 5 |
| Povidone K-12 | 6.3 | 10 |
| Water | 2.0 | 3.2 |
| Total | 100 | 160 |

### EXAMPLE 10:

| **Ingredient** | **(%w/w)** | **mg** |
|---|---|---|
| Loratadine | 6.3 | 10 |
| Capmul™ MCM | 82.4 | 131.8 |
| Polysorbate™ 80 | 3.1 | 5 |
| Povidone K-12 | 6.3 | 10 |
| Water | 2.0 | 3.2 |
| Total | 100 | 160 |

### EXAMPLE 11:

| **Ingredient** | **(%w/w)** | **mg** |
|---|---|---|
| Loratadine | 6.3 | 10 |
| Captex™ 355 | 37.1 | 59.3 |
| Capmul™ MCM | 42.2 | 67.6 |
| Polysorbate™ 80 | 4.2 | 6.6 |
| Povidone K-12 | 7.3 | 11.6 |
| Water | 3.1 | 4.9 |
| Total | 100 | 160 |

### EXAMPLE 12:

| **Ingredient** | **(%w/w)** | **mg** |
|---|---|---|
| Loratadine | 6.3 | 10 |
| Captex™ 355 | 45.3 | 72.5 |
| Capmul™ MCM | 40.1 | 64.2 |
| Polysorbate™ 80 | 2.1 | 3.3 |
| Povidone K-12 | 5.2 | 8.4 |
| Water | 1.0 | 1.6 |
| Total | 100 | 160 |

### EXAMPLE 13:

| **Ingredient** | **(%w/w)** | **mg** |
|---|---|---|
| Loratadine | 6.3 | 10 |
| Captex™ 355 | 42.2 | 67.5 |
| Capmul™ MCM | 37.1 | 59.3 |
| Polysorbate™ 80 | 4.2 | 6.7 |
| Povidone K-12 | 7.2 | 11.6 |
| Water | 3.1 | 4.9 |
| Total | 100 | 160 |

### EXAMPLE 14:

| **Ingredient** | **(%w/w)** | **mg** |
|---|---|---|
| Loratadine | 6.3 | 10 |
| Captex™ 355 | 40.1 | 64.2 |
| Capmul™ MCM | 45.3 | 72.5 |
| Polysorbate™ 80 | 2.1 | 3.3 |
| Povidone K-12 | 5.2 | 8.4 |
| Water | 1.0 | 1.6 |
| Total | 100 | 160 |

### EXAMPLE 15:

| **Ingredient** | **(%w/w)** | **mg** |
|---|---|---|
| Loratadine | 6.3 | 10 |
| Captex™ 355 | 41.3 | 66 |
| Capmul™ MCM | 41.3 | 66 |
| Polysorbate™ 80 | 2.8 | 4.5 |
| Povidone K-12 | 6.3 | 10.1 |
| Water | 2.1 | 3.4 |
| Total | 100 | 160 |

### EXAMPLE 16:

| **Ingredient** | **(%w/w)** | **mg** |
|---|---|---|
| Loratadine | 6.3 | 10 |
| Captex™ 355 | 41.1 | 65.8 |
| Capmul™ MCM | 41.1 | 65.8 |
| Polysorbate™ 80 | 3.4 | 5.5 |
| Povidone K-12 | 6.2 | 9.9 |
| Water | 1.9 | 3 |
| Total | 100 | 160 |

### EXAMPLE 17:

| **Ingredient** | **(%w/w)** | **mg** |
|---|---|---|
| Loratadine | 6.3 | 10 |
| Captex™ 355 | 41.4 | 66.2 |
| Capmul™ MCM | 41.4 | 66.2 |
| Polysorbate™ 80 | 3.3 | 5.2 |
| Povidone K-12 | 5.6 | 9.0 |
| Water | 2.1 | 3.4 |
| Total | 100 | 160 |

### EXAMPLE 18:

| **Ingredient** | **(%w/w)** | **mg** |
|---|---|---|
| Loratadine | 6.3 | 10 |
| Captex™ 355 | 41.0 | 65.7 |
| Capmul™ MCM | 41.0 | 65.7 |
| Polysorbate™ 80 | 2.9 | 4.7 |
| Povidone K-12 | 6.9 | 11 |
| Water | 1.8 | 2.9 |
| Total | 100 | 160 |

### EXAMPLE 19:

| **Ingredient** | **(%w/w)** | **mg** |
|---|---|---|
| Loratadine | 6.3 | 10 |
| Captex™ 355 | 41.3 | 66 |
| Capmul™ MCM | 41.3 | 66 |
| Polysorbate™ 80 | 3.2 | 5.1 |
| Povidone K-12 | 6.3 | 10.1 |
| Water | 1.8 | 2.9 |
| Total | 100 | 160 |

### EXAMPLE 20:

| **Ingredient** | **(%w/w)** | **mg** |
|---|---|---|
| Loratadine | 6.3 | 10 |
| Captex™ 355 | 41.1 | 65.8 |
| Capmul™ MCM | 41.1 | 65.8 |
| Polysorbate™ 80 | 3.1 | 4.9 |
| Povidone K-12 | 6.2 | 9.9 |
| Water | 2.2 | 3.5 |
| Total | 100 | 160 |

### EXAMPLE 21:

| **Ingredient** | **(%w/w)** | **mg** |
|---|---|---|
| Loratadine | 6.3 | 10 |
| Captex™ 355 | 42.2 | 101.3 |
| Capmul™ MCM | 42.2 | 101.4 |
| Polysorbate™ 80 | 3.1 | 7.5 |
| Povidone K-12 | 6.3 | 15 |
| Water | 2.0 | 4.8 |
| Total | 100 | 240 |

### EXAMPLE 22:

| **Ingredient** | **(%w/w)** | **mg** |
|---|---|---|
| Loratadine | 6.3 | 10 |
| Captex™ 355 | 42.5 | 127.6 |
| Capmul™ MCM | 42.5 | 127.5 |
| Polysorbate™ 80 | 3.3 | 10 |
| Povidone K-12 | 6.3 | 18.9 |
| Water | 2.0 | 6.0 |
| Total | 100 | 300 |

### EXAMPLE 23:

### Dissolution Profile of the Loratadine Soft Gel Capsule

The dissolution profile of the formulation described in Example 6, which was encapsulated into a 3 oval soft gelatin capsule, was determined using the USP dissolution apparatus #2 using water at 37° C as the dissolution medium with a paddle speed of 50 RPM.

The release of the drug was determined by HPLC using a UV detector at 272 nm.

The dissolution results are presented in the table below.

| **TIME** | **% LORATADINE** |
|---|---|
| 10 minutes | 102% |
| 15 minutes | 103% |
| 20 minutes | 103% |
| 30 minutes | 103% |
| 45 minutes | 103% |

In view of the teachings presented herein, other modifications and variations of the present inventions will be readily apparent to those of skill in the art. The foregoing discussion and description are illustrative of some embodiments of the present invention, but are not meant to be limitations on the practice thereof. Accordingly, all suitable modifications, variations and equivalents may be resorted to, and such modifications, variations and equivalents are intended to fall within the scope of the invention as described and within the scope of the claims.

The present invention is further illustrated by the following items:
1. A pharmaceutical formulation of loratadine, comprising:
   loratadine;
   one or more mono- and di- glyceride medium chain fatty acids;
   one or more dispersants; and
   one or more surfactants.
2. A formulation according to claim 1, further comprising one or more triglyceride medium chain fatty acids.
3. A formulation according to any one of items 1 to 2, further comprising water.
4. A formulation according to any one of items 1 to 3, wherein the amount of loratadine ranges from about 3% to about 7% by weight.
5. A formulation according to any one of items 1 to 4, wherein the amount of mono- and di- glyceride medium chain fatty acids ranges from about 33% to about 83% by weight.
6. A formulation according to any one of items 2 to 5, wherein the amount of triglyceride medium chain fatty acids ranges from about 8% to about 50% by weight.
7. A formulation according to any one of items 1 to 6, wherein the amount of dispersants ranges from about 7% to about 12% by weight.
8. A formulation according to any one of items 1 to 7, wherein the amount of surfactants ranges from about 2% to about 5% by weight.
9. A formulation according to any one of items 3 to 7, wherein the amount of water ranges from about 1% to about 4% by weight.
10. A formulation according to item 1, wherein the mono- and di- glyceride medium chain fatty acids are selected from the group consisting of glyceryl mono- & dicaprate, propylene glycol monocaprylate, propylene glycol caprylate, propylene glycol laurate and propylene glycol monolaurate.
11. A formulation according to item 2, wherein the triglyceride medium chain fatty acids are selected from the group consisting of glyceryl tricaprylate/caprate, and glycerol caprylate caprate and caprylic/capric triglycerides.
12. A formulation according to item 1, wherein the dispersants include povidone.
13. A formulation according to item 12, wherein the povidone is selected from the group consisting of povidone K-12, povidone K-17 and povidone K-30.
14. A formulation according to item 1, wherein the surfactants include a non-ionic surfactant.
15. A formulation according to item 14, wherein the surfactants are selected from the group consisting of polyoxyethylene sorbitan fatty acid esters, ethoxylated aliphatic alcohols, and caprylocaproyl macrogol-8 glycerides.
16. A formulation according to item 1, wherein the amount of dispersants to amount of surfactants are present in a ratio of about 1.75:1 to about 2.5:1.
17. A formulation according to item 1, wherein the formulation has a hydrophilic lipophilic balance (HLB) value between about 3 and about 7.
18. A formulation according to item 17, wherein the HLB value is between about 5 and about 6.
19. A formulation according to item 1, wherein the monoglyceride medium fatty acids have a HLB value between about 5 and about 6.
20. A formulation according to item 2, wherein the triglyceride medium chain fatty acids have a HLB value of about 11.
21. A formulation according to item 1, wherein the surfactant has a HLB value between about 14 and about 17.
22. A loratadine dosage form for oral administration, comprising:
   a drug delivery vehicle; and
   a fill formulation comprising loratadine, one or more mono- and di- glyceride medium chain fatty acids, one or more dispersants, and one or more surfactants disposed within the drug delivery vehicle.
23. A dosage form according to item 22, further comprising one or more triglyceride medium chain fatty acids.
24. A dosage form according to any one of items 22 to 23, further comprising water.
25. A dosage form according to any one of items 22 to 24, wherein the amount of loratadine ranges from about 3% to about 7% by weight.
26. A dosage form according to any one of items 22 to 25, wherein the amount of mono- and di- glyceride medium chain fatty acids ranges from about 33% to about 83% by weight.
27. A dosage form according to any one of items 22 to 26, wherein the amount of triglyceride medium chain fatty acids ranges from about 8% to about 50% by weight.
28. A dosage form according to any one of items 22 to 27, wherein the amount of dispersants ranges from about 7% to about 12% by weight.
29. A dosage form according to any one of items 22 to 28, wherein the amount of surfactants ranges from about 2% to about 5% by weight.
30. A dosage form according to any one of items 22 to 29, wherein the amount of water ranges from about 1% to about 4% by weight.

## Claims

1. A pharmaceutical formulation of loratadine, comprising:
loratadine;
one or more mono- and di- glyceride medium chain fatty acids;
one or more dispersants; and
one or more surfactants.

2. A formulation according to claim 1, further comprising one or more triglyceride medium chain fatty acids, wherein the amount of triglyceride medium chain fatty acids preferably ranges from about 8% to about 50% by weight and/or wherein the triglyceride medium chain fatty acids preferably are selected from the group consisting of glyceryl tricaprylate/caprate, and glycerol caprylate caprate and caprylic/capric triglycerides.

3. A formulation according to any one of claims 1 to 2, further comprising water, preferably wherein the amount of water ranges from about 1% to about 4% by weight.

4. A formulation according to any one of claims 1 to 3, wherein the amount of loratadine ranges from about 3% to about 7% by weight.

5. A formulation according to any one of claims 1 to 4, wherein the amount of mono- and di- glyceride medium chain fatty acids ranges from about 33% to about 83% by weight.

6. A formulation according to any one of claims 1 to 6, wherein the amount of dispersants ranges from about 7% to about 12% by weight and/or wherein the amount of surfactants ranges from about 2% to about 5% by weight.

7. A formulation according to claim 1, wherein the mono- and di- glyceride medium chain fatty acids are selected from the group consisting of glyceryl mono- & dicaprate, propylene glycol monocaprylate, propylene glycol caprylate, propylene glycol laurate and propylene glycol monolaurate.

8. A formulation according to claim 1, wherein the dispersants include povidone, preferably wherein the povidone is selected from the group consisting of povidone K-12, povidone K-17 and povidone K-30.

9. A formulation according to claim 1, wherein the surfactants include a non-ionic surfactant, preferably wherein the surfactants are selected from the group consisting of polyoxyethylene sorbitan fatty acid esters, ethoxylated aliphatic alcohols, and caprylocaproyl macrogol-8 glycerides.

10. A formulation according to claim 1, wherein the amount of dispersants to amount of surfactants are present in a ratio of about 1.75:1 to about 2.5:1.

11. A formulation according to claim 1, wherein the formulation has a hydrophilic lipophilic balance (HLB) value between about 3 and about 7, preferably wherein the HLB value is between about 5 and about 6.

12. A formulation according to claim 1, wherein the monoglyceride medium fatty acids have a HLB value between about 5 and about 6 and/or wherein the triglyceride medium chain fatty acids have a HLB value of about 11.

13. A formulation according to claim 1, wherein the surfactant has a HLB value between about 14 and about 17.

14. A loratadine dosage form for oral administration, comprising:
a drug delivery vehicle; and
a fill formulation according to any one of claims 1 to 13 disposed within the drug delivery vehicle.

15. A dosage form according to claim 22, wherein the drug delivery vehicle comprises a capsule, preferably wherein the capsule comprises a soft gelatin capsule or consists essentially of gelatin.
